# EUROPEAN PATENT APPLICATION

(11) **EP 4 666 935 A1**
(43) Date of publication of application: **24.12.2025**
(21) Application number: 24785026.6
(22) Date of filing: 06.02.2024
(51) Int. Cl.: A61B 5/00, A61B 5/1455, A61B 5/024, A61B 5/026

(54) **WEARABLE DEVICE COMPRISING SENSOR**

(30) Priority: 04.04.2023 KR 20230044423; 20.04.2023 KR 20230052287
(71) Applicant: Samsung Electronics Co., Ltd., Suwon-si, Gyeonggi-do 16677 (KR)
(72) Inventor: PARK, Sangkyung, Suwon-si Gyeonggi-do 16677 (KR); SEOK, Munki, Suwon-si Gyeonggi-do 16677 (KR); RHEE, Daesun, Suwon-si Gyeonggi-do 16677 (KR)
(74) Representative: Gulde & Partner
(86) International application number: PCT/KR2024/001771
(87) International publication number: WO 2024/210315

(57) **Abstract**

A wearable device according to one embodiment may comprise a housing including: a first surface facing a part of the body of a user when the wearable device is worn on the user; and a second surface opposite to the first surface. The wearable device may comprise a sensor module which is arranged between the first surface and the second surface and which includes a light-emitting unit and a light-receiving unit spaced apart from the light-emitting unit. The housing can include a groove, which is arranged between the light-emitting unit and the light-receiving unit and which is indented from the first surface toward the second surface.

## Description

### [Technical Field]

Various embodiments to be described later relate to a wearable device including a sensor.

### [Background Art]

A wearable device may be used in a state of being worn on a part of a body of a user. The wearable device may be provided as various types of products. For example, the wearable device may include a ring shaped device for the user to be worn on a part of the body of the user. The wearable device may provide various user experiences to the user by including a sensor for providing user-related information.

### [Disclosure]

### [Technical Solution]

A wearable device according to an embodiment may include a housing including a first side facing a part of a body of a user when the wearable device is worn by the user, and a second side opposite to the first side. The wearable device may include a sensor module disposed between the first side and the second side, the sensor module including a light emitting unit and a light receiving unit spaced apart from the light emitting unit. The housing may include a groove disposed between the light emitting unit and the light receiving unit and dent toward the second side from the first side.

A wearable device according to an embodiment may include a housing including a first side facing a part of a body of a user when the wearable device is worn by the user, and a second side opposite to the first side. The wearable device may include a hole formed by the second side to pass through the part of the body of the user. The wearable device may include a sensor module disposed between the first side and the second side, the sensor module including a light emitting unit and a light receiving unit spaced apart from the light emitting unit. The wearable device may include a processor operatively coupled to the sensor module. The housing may include a groove disposed between the light emitting unit and the light receiving unit and dent toward the second side from the first side to reduce transmission to the light receiving unit of light emitted from the light emitting unit toward the light receiving unit. The processor may be configured to emit light toward the part of the body of the user on which the wearable device is worn, using the light emitting unit of the sensor module, and obtain biometric information of the user by receiving at least a portion of the light reflected by the part of the body using the light receiving unit of the sensor module.

### [Description of the Drawings]

FIG. 1 is a block diagram of an electronic device in a network environment according to an embodiment.
FIG. 2A illustrates an exemplary electronic device.
FIG. 2B is an exploded perspective view of an exemplary electronic device.
FIGS. 2C and 2D are partial cross-sectional views of an exemplary electronic device.
FIGS. 3A and 3B are partial cross-sectional views of an exemplary electronic device.
FIG. 4A is a perspective view of an exemplary electronic device.
FIG. 4B is a partial cross-sectional view of the exemplary electronic device of FIG. 4A.
FIG. 5A is a perspective view of an exemplary electronic device.
FIGS. 5B and 5C are partial cross-sectional views of the exemplary electronic device of FIG. 5A.

### [Mode for Invention]

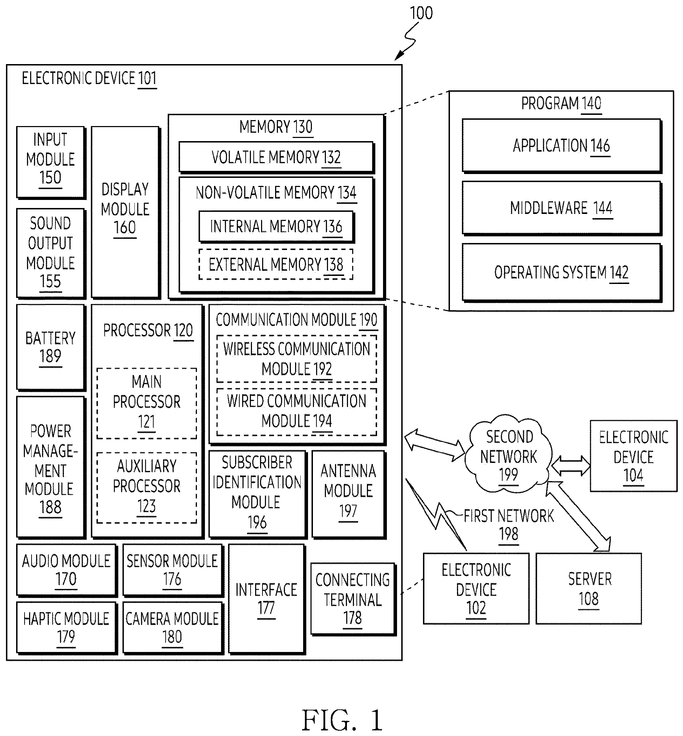

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

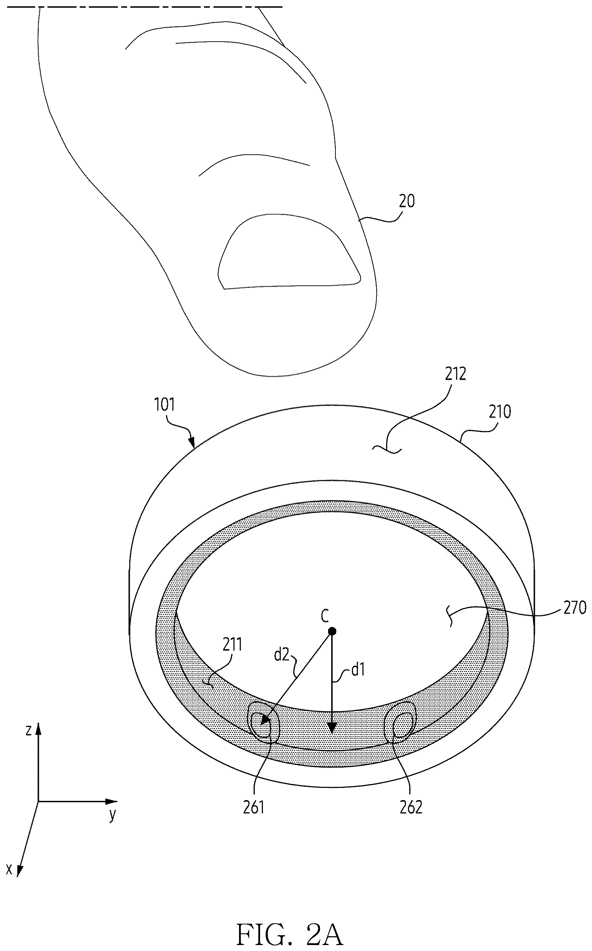

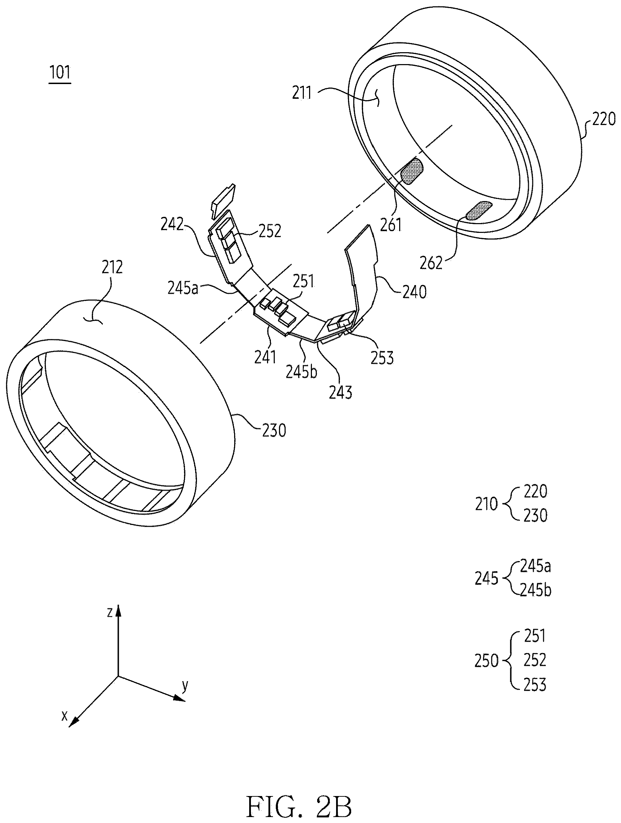

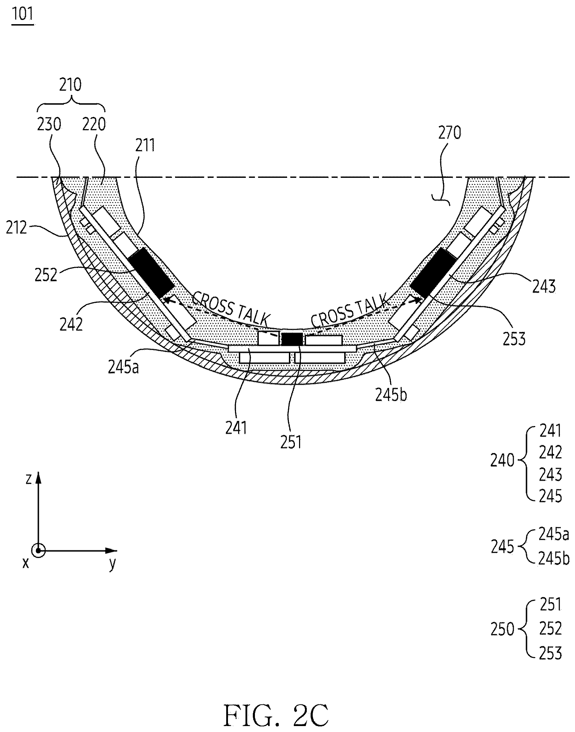

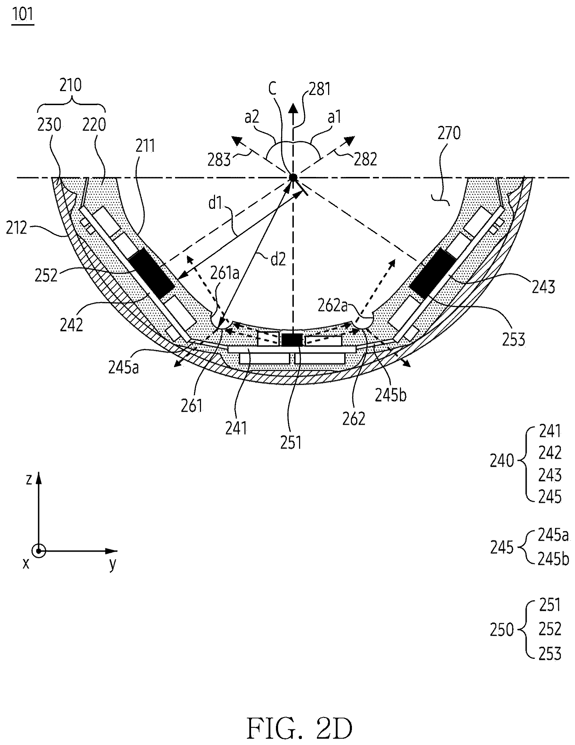

FIG. 2A illustrates an exemplary electronic device. FIG. 2B is an exploded perspective view of an exemplary electronic device. FIGS. 2C and 2D are partial cross-sectional views of an exemplary electronic device.

Referring to FIGS. 2A, 2B, 2C, and 2D, an electronic device 101 may include a housing 210, and a sensor module 250 (e.g., the sensor module 176 of FIG. 1).

According to an embodiment, the electronic device 101 may be referred to as a wearable device that may be worn by a user. The user may mean a person who wears the electronic device 101. The electronic device 101 may be worn by a part 20 of a body of the user. For example, the electronic device 101 may be worn on the part 20 of the body of the user. For example, the electronic device 101 may be fastened to the part 20 of the body of the user. For example, the electronic device 101 may be detachable from the part 20 of the body of the user.

For example, the electronic device 101 may be in contact with the part 20 of the body of the user by being worn by the user. For example, the electronic device 101 may be configured to obtain information associated with the user through the part 20 of the body of the user by being worn by the user. For example, the electronic device 101 may provide the information indicating a state of the user to the user based on obtaining the information associated with the user. For example, the electronic device 101 may provide the information indicating the state of the user to the user by being configured to display the information indicating the state of the user through a display module (e.g., the display module 160 of FIG. 1) of the electronic device 101 and/or an external electronic device (e.g., the electronic device 102 or the electronic device 104 of FIG. 1) connected with the electronic device 101. However, it is not limited thereto.

For example, the part 20 of the body of the user on which the electronic device 101 is worn may be a finger of the user. For example, the housing 210 of the electronic device 101 may have a ring shape so that the electronic device 101 is worn on the finger of the user. However, it is not limited thereto. The electronic device 101 that may be referred to as the wearable device may have a shape corresponding to the part 20 of the body in order to be worn on the part 20 of the body of the user.

According to an embodiment, the housing 210 may include a first side 211 facing the part 20 of the body of the user when the electronic device 101 is worn by the user, and a second side 212 opposite to the first side 211.

For example, in a case that the electronic device 101 is worn by the user, at least a portion of the first side 211 may be in contact with the part 20 of the body of the user. For example, the first side 211 may surround the part 20 of the body of the user on which the electronic device 101 is worn. For example, the first side 211 may cover the part 20 of the body of the user on which the electronic device 101 is worn. For example, the first side 211 may be configured so that the electronic device 101 is fastened to the part 20 of the body, by pressurizing the part 20 of the body of the user when the electronic device 101 is worn by the user. For example, the first side 211 may be deformable by the part 20 of the body of the user. For example, the electronic device 101 may provide a haptic technology to transmit the information associated with the state of the user through the first side 211 in a case that the electronic device 101 is worn by the user.

For example, the second side 212 may form an exterior of the electronic device 101 together with the first side 211. For example, the second side 212 may form the ring-shaped housing 210 together with the first side 211. For example, the second side 212 may be a surface spaced apart from the part 20 of the body of the user in a case that the electronic device 101 is worn by the user. For example, when the electronic device 101 is worn by the user, the first side 211 may be a side that is closest to the part 20 of the body of the user. The second side 212 opposite to the first side 211 may be a side that is farthest from the part 20 of the body of the user. For example, the first side 211 may be referred to as an inner circumference surface of the housing 210. The second side 212 opposite to the first side 211 may be referred to as an outer circumference surface of the housing 210.

According to an embodiment, the electronic device 101 may further include a hole 270 formed by the first side 211 to pass through the part 20 of the body of the user when the electronic device 101 is worn by the user. For example, the first side 211 may define the hole 270. For example, in a case that the electronic device 101 is worn by the user, the hole 270 may be penetrated by the part 20 of the body of the user. The electronic device 101 may be configured to be fastened to the part 20 of the body of the user in a case that the user wears the electronic device 101, by including the hole 270 configured to pass through the part 20 of the body of the user.

According to an embodiment, the sensor module 250 may be disposed between the first side 211 and the second side 212. The sensor module 250 may include a light emitting unit 251 and at least one or more light receiving unit (e.g., a light receiving unit 252 and another light receiving unit 253) spaced apart from the light emitting unit 251.

For example, the sensor module 250 may be disposed in an inner space of the housing 210 between the first side 211 and the second side 212. For example, the sensor module 250 may be disposed on an element (e.g., a flexible printed circuit board 240) of the electronic device 101 between the first side 211 and the second side 212. The sensor module 250 may be electrically connected with the element. For example, the sensor module 250 may be configured to sense the state of the user using the part 20 of the body of the user on which the electronic device 101 is worn. The electronic device 101 may be configured to provide the user with the information associated with the state through the detected state of the user. The sensor module 250 may include at least one sensor. For example, the sensor module 250 may include at least one of an optical sensor or a heartrate measurement (HRM) sensor using a photoplethysmography (PPG), but is not limited thereto. The light emitting unit 251 may be referred to as a light emitting diode (LED), and the light receiving unit 252 may be referred to as a photo diode, but is not limited thereto.

For example, the light emitting unit 251 may be configured to emit light in a plurality of directions. A portion of light emitted from the light emitting unit 251 in the plurality of directions may be reflected by the part 20 of the body of the user on which the electronic device 101 is worn. For example, the light emitting unit 251 may be configured to emit light toward the part 20 of the body of the user on which the electronic device 101 is worn. The light emitted from the light emitting unit 251 toward the part 20 of the body of the user may be reflected by the part 20 of the body. According to an embodiment, a portion of light emitted from the light emitting unit 251 in the plurality of directions may be configured to be transmitted toward the light receiving unit 252. For example, the light transmitted from the light emitting unit 251 to the light receiving unit 252 may be transmitted to the light receiving unit 252 through an element (e.g., a first frame 220 of FIG. 2B) of the housing 210.

For example, the light receiving unit 252 may be configured to receive a portion of light emitted in the plurality of directions from the light emitting unit 251. For example, the light emitting unit 251 may be configured to emit light toward the part 20 of the body of the user on which the electronic device 101 is worn. According to an embodiment, the light receiving unit 252 may be configured to receive a portion of light reflected by the part 20 of the body of the user. For example, the light receiving unit 252 may be configured to receive the portion of light emitted by the light emitting unit 251 in the plurality of directions. The light receiving unit 252 may be configured so that the light emitting unit 251 receives a portion of light emitted in the plurality of directions through a space and/or a medium between the first side 211 and the second side 212 of the housing 210.

According to an embodiment, the sensor module 250 may be configured to detect the state of the user based on light emitted from the light emitting unit 251 and reflected by the part 20 of the body of the user being received by the light receiving unit 252. The electronic device 101 may be configured to obtain the information associated with the state of the user from the sensor module 250. In the sensor module 250, since the portion of light emitted from the light emitting unit 251 is directly received by the light receiving unit 252, cross talk may occur in association with detecting the state of the user through the part 20 of the body of the user. For example, when referring to FIG. 2C, the portion of light emitted from the light emitting unit 251 may not be reflected from the part 20 of the body, but may be transmitted to the light receiving unit 252 through the element (e.g., the first frame 220 of FIG. 2B) of the housing 210 or directly. The electronic device 101 may be required to reduce the cross talk in order to provide the user with the information indicating the state of the user.

According to an embodiment, the housing 210 may include a groove 261 disposed between the light emitting unit 251 and the light receiving unit 252 and dent toward the second side 212 from the first side 211.

In this document, when an element is mentioned as being "between" other elements, it should be noted that a disposition relationship between the three elements are not limited, since not only it includes a relationship that the element and the other elements are disposed on the same flat surface, but also it includes that the element is disposed on a virtual line (e.g., a straight line or a curved line) connecting the other elements in a state that the element and each of the other elements are disposed on different flat surfaces. For example, "the groove 261 between the light emitting unit 251 and the light receiving unit 252" may indicate "the groove 261 disposed on a virtual line connecting the light emitting unit 251 and the light receiving unit 252". For example, "the groove 261 between the light emitting unit 251 and the light receiving unit 252" may indicate "the groove 261 disposed on a path of light emitted from the light emitting unit 251 toward the light receiving unit 252".

For example, the groove 261 may extend from the first side 211 toward the second side 212. For example, the groove 261 may be formed on the first side 211. For example, the groove 261 may separate the light emitting unit 251 and the light receiving unit 252. For example, the groove 261 may be formed in a medium between the light emitting unit 251 and the light receiving unit 252. For example, by having an inner surface between the light emitting unit 251 and the light receiving unit 252, the groove 261 may be formed so that the medium through which the light emitted from the light emitting unit 251 is transmitted is different based on the inner surface. The inner surface of the groove 261 may be an interface at which the medium is different based on the inner surface. For example, since the medium is different based on the inner surface of the groove 261, the groove 261 may be disposed so that light emitted from the light emitting unit 251 toward the inner surface of the groove 261 is refracted and/or reflected based on the inner surface of the groove 261.

For example, the groove 261 may be disposed on the path of the light emitted from the light emitting unit 251 toward the light receiving unit 252. As the groove 261 is formed on the path of the light emitted from the light emitting unit 251 toward the light receiving unit 252, the groove 261 may refract light emitted directly from the light emitting unit 251 toward the light receiving unit 252. The groove 261 may refract the light emitted directly from the light emitting unit 251 toward the light receiving unit 252, thereby reducing cross talk as the light receiving unit 252 receives light not reflected by the part 20 of the body of the user.

According to an embodiment, the inner surface of the groove 261 may include a curved surface 261a extending from the first side 211 and bending to have a curvature.

For example, the inner surface of the groove 261 may extend from the first side 211 toward the second side 212 opposite to the first side 211. For example, the inner surface of the groove 261 may have a convex shape toward the second side 212. For example, the inner surface of the groove 261 may have a concave shape with respect to the first side 211. For example, the inner surface of the groove 261 may be formed so that an incline at a point of the curved surface 261a included in the inner surface is different from an incline at another point of the curved surface 261a different from the point. For example, the groove 261 may have a dome shape, but is not limited thereto.

For example, since the groove 261 is disposed on the path through which the light emitted from the light emitting unit 251 is directly transmitted to the light receiving unit 252, the light transmitted directly from the light emitting unit 251 to the light receiving unit 252 may be transmitted to the inner surface of the groove 261. Since the inner surface of the groove 261 includes the curved surface 261a extending from the first side 211 and bending to have the curvature, light transmitted from the light emitting unit 251 to the inner surface of the groove 261 may be refracted and/or reflected in a plurality of directions by the curved surface 261a. Since the light is refracted and/or reflected in the plurality of directions, the groove 261 may reduce cross talk as the light emitted from the light emitting unit 251 toward the light receiving unit 252 is received by the light receiving unit 252.

According to an embodiment, a distance d1 from a center C of the hole 270 to the first side 211 may be shorter than a distance d2 from the center C to the groove 261. The center C of the hole 270 may be a point at which vertical distances from the first side 211 to the center C are each substantially the same in a case that the hole 270 has a circular shape. The center C of the hole 270 may be a point at which a major axis of the hole 270 and a minor axis of the hole 270 cross each other in a case that the hole 270 has an oval shape. For example, the distance d2 from the center C of the hole 270 to a point on the inner surface of the groove 261 may be longer than the distance d1 from the center C to the first side 211. Since the distance d1 is shorter than the distance d2, the electronic device 101 may reduce the cross talk as the light emitted from the light emitting unit 251 is directly received by the light receiving unit 252.

According to an embodiment, an angle al between a first direction 281 from the light emitting unit 251 toward the center C of the hole 270 and the second direction 282 from the light receiving unit 252 toward the center C is within a range of 45 degrees or more and 55 degrees or less. For example, the light receiving unit 252 may be inclined with respect to the light emitting unit 251 in the housing 210 so that the angle a1 between the first direction 281 and the second direction 282 is within the range of 45 degrees or more and 55 degrees or less. As the angle between the first direction 281 and the second direction 282 is within the range of 45 degrees or more and 55 degrees or less, the electronic device 101 may increase a probability that the light emitted from the light emitting unit 251 toward the part 20 of the body of the user on which the electronic device 101 is worn and reflected by the part 20 of the body is received by the light receiving unit 252.

According to an embodiment, the sensor module 250 may further include the other light receiving unit 253 faced away from the light receiving unit 252 and spaced apart from the light emitting unit 251. The housing 210 may further include another groove 262 disposed between the light emitting unit 251 and the other light receiving unit 253 and dent toward the second side 212 from the first side 211. For example, the light receiving unit 252 may be positioned in a -y direction with respect to the light emitting unit 251 based on an x-axis. The other light receiving unit 253 may be positioned in a +y direction with respect to the light emitting unit 251 based on the x-axis. For example, the light emitting unit 251 may be disposed between the light receiving unit 252 and the other light receiving unit 253. The other light receiving unit 253 may be configured substantially the same as or similar to the light receiving unit 252.

For example, the other groove 262 may be formed substantially the same as or similar to the groove 261. For example, the other groove 262 may be disposed on a path through which light emitted from the light emitting unit 251 is directly transmitted to the other light receiving unit 253. The other groove 262 may refract the emitted light directly transmitted from the light emitting unit 251 to the other light receiving unit 253 by being formed on the path through which the light emitted from the light emitting unit 251 is directly transmitted to the other light receiving unit 253. The groove 261 may refract the emitted light so that the light emitted from the light emitting unit 251 is not directly transmitted to the other light receiving unit 253, thereby reducing cross talk as the other light receiving unit 253 receives light not reflected by the part 20 of the body of the user. For example, an inner surface of the other groove 262 may include a curved surface 262a extending from the first side 211 and bending to have a curvature. Since the inner surface of the other groove 262 includes the curved surface 262a extending from the first side 211 and bending to have the curvature, light transmitted from the light emitting unit 251 to the inner surface of the other groove 262 may be refracted and/or reflected in a plurality of directions by the curved surface 262a.

It has been described that the sensor module 250 of the electronic device 101 includes the light receiving unit 252 and the other light receiving unit 253, but is not limited thereto. The sensor module 250 of the electronic device 101 may include a plurality of light receiving units for receiving light emitted from the light emitting unit 251 and reflected from the part 20 of the body of the user on which the electronic device 101 is worn. The housing 210 may include a plurality of grooves respectively disposed between the light emitting unit 251 and the plurality of light receiving units and dent toward the second side 212 from the first side 211 to reduce cross talk as the light emitted from the light emitting unit 251 is directly transmitted to the plurality of light receiving units.

According to an embodiment, the angle a1 between the first direction 281 from the light emitting unit 251 toward the center C of the hole 270 and the second direction 282 from the light receiving unit 252 toward the center C may correspond to an angle a2 between the first direction 281 from the light emitting unit 251 toward the center C and a third direction 283 from the other light receiving unit 252 toward the center C. For example, each of the light receiving unit 252 and the other light receiving unit 253 may be disposed to be respectively inclined with respect to the light emitting unit 251 in the housing 210. For example, the angle a1 between the first direction 281 and the second direction 282 may be substantially the same as the angle a2 between the first direction 281 and the third direction 283. As the angle a1 corresponds to the angle a2, the electronic device 101 may increase a probability that the light emitted from the light emitting unit 251 toward the part 20 of the body of the user on which the electronic device 101 is worn and the light reflected by the part 20 of the body is received by the light receiving unit 252 or the other light receiving unit 253. According to an embodiment, the angle a2 may be within a range of 45 degrees or more and 55 degrees or less.

According to an embodiment, the housing 210 may further include the first frame 220 and a second frame 230. The first frame 220 may define the first side 211 and may be in contact with the part 20 of the body of the user. The second frame 230 may define the second side 212 and may be coupled with the first frame 220.

For example, the first frame 220 may be a portion of the housing 210 including the first side 211. For example, in a case that the electronic device 101 is worn by the user, the first frame 220 may be in contact with the part 20 of the body of the user. For example, the first frame 220 may be pressurized by the part 20 of the body of the user. For example, in the first frame 220, the groove 261 (and/or the other groove 262) dent toward the second side 212 from the first side 211 may be formed. Since the groove 261 is disposed between the light emitting unit 251 and the light receiving unit 252 of the sensor module 250, the first frame 220 that provides a medium to the light emitted from the light emitting unit 251 toward the light receiving unit 252 may reduce cross talk as the light is received by the light receiving unit 252, by refracting the light by the groove 261. For example, the first frame 220 may include at least one of epoxy and rubber. The epoxy transmits the light emitted from the light emitting unit 251, and the rubber may block the light emitted from the light emitting unit 251 and directly facing the light receiving unit 252, but is not limited thereto.

For example, the second frame 230 may surround the first frame 220. For example, the second frame 230 may support the first frame 220. For example, the second frame 230 may form an exterior of the housing 210 together with the first frame 220. For example, the second frame 230 may be a portion of the housing 210 including the second side 212 opposite to the first side 211. The second frame may include a metal material, but is not limited thereto.

According to an embodiment, the electronic device 101 may further include the flexible printed circuit board 240 disposed between the first side 211 and the second side 212 and connected with the sensor module 250. The flexible printed circuit board 240 may include a first support portion 241 supporting the light emitting unit 251, a second support portion 242 spaced apart from the first support portion 241 and supporting the light receiving unit 252, and a first connection portion 245a connecting the first support portion 241 and the second support portion 242.

For example, the flexible printed circuit board 240 may be electrically connected with the sensor module 250. For example, the flexible printed circuit board 240 may be disposed in the housing 210. The flexible printed circuit board 240 may include a bent part corresponding to a shape of the housing 210. For example, the flexible printed circuit board 240 may be connected to a plurality of electronic components. For example, the flexible printed circuit board 240 may be disposed between the first frame 220 and the second frame 230. For example, the printed circuit board 240 may include a rigid flexible printed circuit board (RFPCB), but is not limited thereto.

For example, the first support portion 241 and the second support portion 242 may be attached on the second frame 230. The second support portion 242 may be inclined with respect to the first support portion 241 so that the light receiving unit 252 receives the light emitted from the light emitting unit 251 and reflected by the part 20 of the body of the user on which the electronic device 101 is worn.

For example, the flexible printed circuit board 240 may further include a third support portion 243 supporting the other light receiving unit 253. The third support portion 243 may be attached on the second frame 230. The third support portion 243 may be faced away from the first support portion 241. However, it is not limited thereto, and the flexible printed circuit board 240 may include a plurality of support portions in which the plurality of electronic components including the sensor module 250 are disposed, respectively.

For example, a connection portion 245 may connect the support portions 241, 242, and 243 of the flexible printed circuit board 240 to each other. For example, by connecting the first support portion 241 and the second support portion 242, the first connection portion 245a may electrically connect an electronic component (e.g., the light emitting unit 251) disposed on the first support portion 241 and an electronic component (e.g., the light receiving unit 252) disposed on the second support portion 242. For example, by connecting the first support portion 241 and the third support portion 243, the second connection portion 245b may electrically connect the electronic component disposed on the first support portion 241 and an electronic component (e.g., the other light receiving unit 243) disposed on the third support portion 243.

According to an embodiment, the electronic device 101 may further include a processor (e.g., the processor 120 of FIG. 1) operatively coupled to the sensor module 250. The processor 120 may be configured to emit light toward the part 20 of the body of the user on which the electronic device 101 is worn using the light emitting unit 251 of the sensor module 250. The processor 120 may be configured to obtain biometric information of the user by receiving at least a portion of the light reflected by the part 20 of the body using the light receiving unit 252 of the sensor module 250.

For example, the processor 120 may be configured to emit light from the light emitting unit 251 of the sensor module 250 based on a user input for indicating biometric information of the electronic device 101. The light emitted from the light emitting unit 251 may be reflected by the part 20 of the body of the user on which the electronic device 101 is worn. The light reflected by the part 20 of the body of the user may be received by the light receiving unit 252 of the sensor module 250. The light receiving unit 252 may detect the biometric information of the user through the part 20 of the body using the light reflected by the part 20 of the body of the user. The processor 120 may be configured to provide the user with the biometric information detected through the light receiving unit 252.

The electronic device 101 according to the above-described embodiment may be configured to provide the user with information associated with the user, using the light emitted from the light emitting unit 251 and reflected by the part 20 of the body of the user on which the electronic device 101 is worn being received by the light receiving unit 252 by including the sensor module 250. The electronic device 101 may include the groove 261 between the light emitting unit 251 and the light receiving unit 252, thereby reducing the cross talk as the light emitted from the light emitting unit 251 is directly received by the light receiving unit 252.

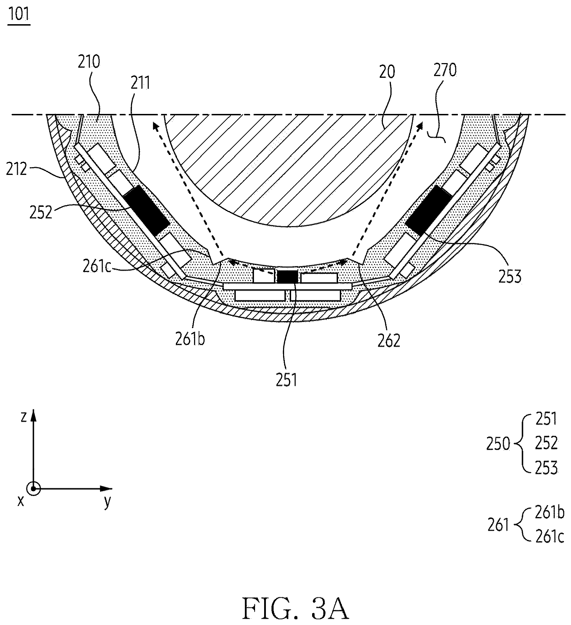

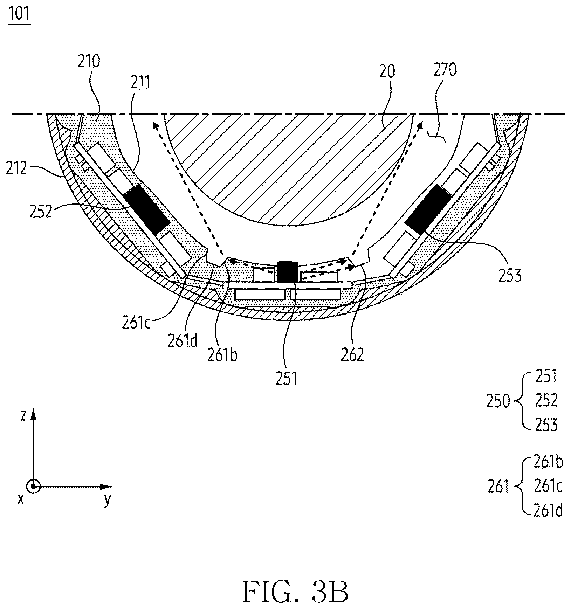

FIGS. 3A and 3B are partial cross-sectional views of an exemplary electronic device.

Referring to FIGS. 3A and 3B, an electronic device 101 may include a housing 210 including a first side 211 facing a part (e.g., the part 20 of the body of FIG. 2A) of a body of a user when the electronic device 101 is worn by the user, and a second side 212 opposite to the first side 211. The electronic device 101 may include a sensor module 250 disposed between the first side 211 and the second side 212 and including a light emitting unit 251 and a light receiving unit 252 spaced apart from the light emitting unit 251. The housing 210 may include a groove 261 disposed between the light emitting unit 251 and the light receiving unit 252 and dent toward the second side 212 from the first side 211.

Hereinafter, an overlapping description of a configuration described in FIGS. 2A, 2B, and 2C will be omitted.

Referring to FIGS. 3A and 3B, an inner surface of the groove 261 may include at least one inclined surface extending from the first side 211 and inclined with respect to an edge of the groove 261, unlike illustrated in FIG. 2C.

For example, when referring to FIG. 3A, the inner surface of the groove 261 may include a first inclined surface 261b and a second inclined surface 261c extending from the first side 211. Since a medium through which light emitted from the light emitting unit 251 is transmitted is different based on the first inclined surface 261b and the second inclined surface 261c, the light emitted from the light emitting unit 251 and transmitted to the first inclined surface 261b and/or the second inclined surface 261c may be refracted when passing through the first inclined surface 261b and/or the second inclined surface 261c or reflected by the first inclined surface 261b and/or the second inclined surface 261c. For example, since the groove 261 is disposed on a path through which light emitted from the light emitting unit 251 is directly transmitted to the light receiving unit 252, the groove 261 including the first inclined surface 261b and the second inclined surface 262c may reduce cross talk as the light receiving unit 252 receives light not reflected by the part 20 of the body of the user by refracting or reflecting the light.

For example, the sensor module 250 may further include another light receiving unit 253. The housing 210 may further include another groove 262 between the light emitting unit 251 and the other light receiving unit 253. The other groove 262 may have at least one inclined surface substantially the same as or similar to the groove 261 to reduce transmission of light from the light emitting unit 251 to the other light receiving unit 253.

For example, when referring to FIG. 3B, the inner surface of the groove 261 may further include a third inclined surface 261d connecting the first inclined surface 261b and the second inclined surface 261c. The inner surface of the groove 261 may widen an inner space of the groove 261 by including the third inclined surface 261d. As the inner space of the groove 261 widens, the groove 261 may reduce the light emitted from the light emitting unit 251 from being directly received by the light receiving unit 252.

It has been described that the groove 261 includes the first inclined surface 261b, the second inclined surface 261c, and the third inclined surface 261d, but is not limited thereto. The groove 261 may include a plurality of inclined surfaces connected to each other to reduce light emitted from the light emitting unit 251 toward the light receiving unit 252 from being received by the light receiving unit 252. Through the plurality of inclined surfaces, the electronic device 101 may reduce cross talk as the light receiving unit 252 receives light not reflected by the part 20 of the body of the user on which the electronic device 101 is worn.

The electronic device 101 according to the above-described embodiment may include the groove 261 disposed between the light emitting unit 251 and the light receiving unit 252 and having the at least one inclined surface, thereby reducing the cross talk as the light receiving unit 252 receives the light not reflected by the part 20 of the body of the user on which the electronic device 101 is worn.

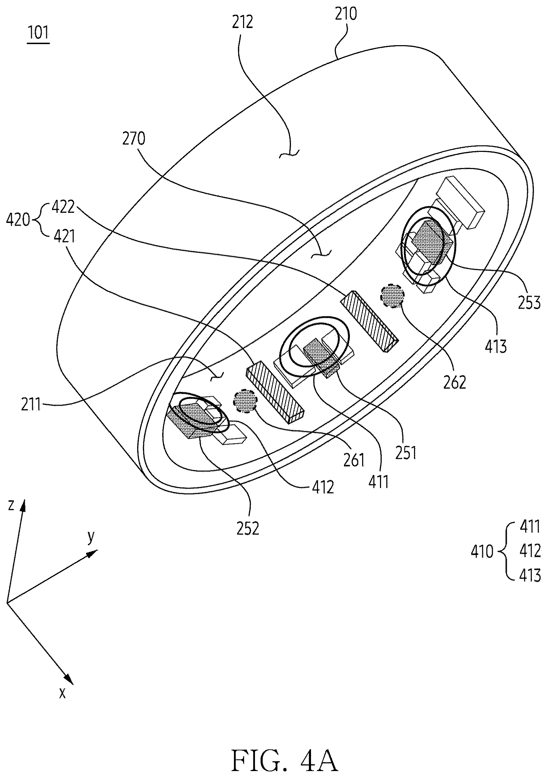

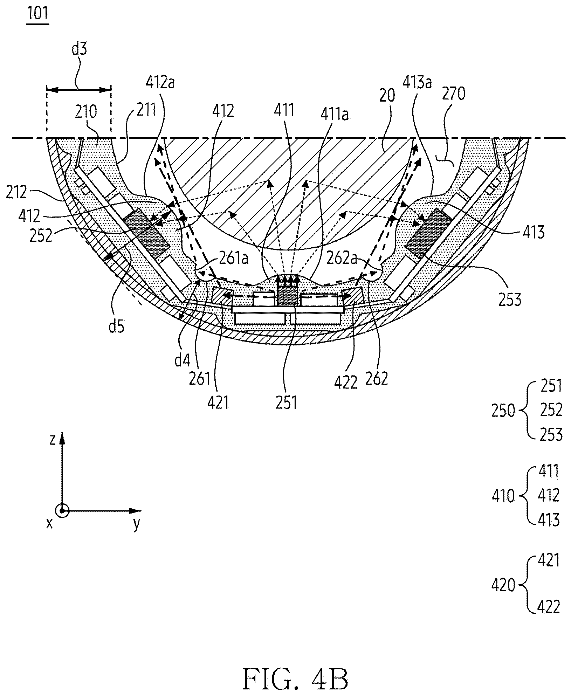

FIG. 4A is a perspective view of an exemplary electronic device. FIG. 4B is a partial cross-sectional view of the exemplary electronic device of FIG. 4A.

Referring to FIGS. 4A and 4B, an electronic device 101 may include a housing 210 including a first side 211 facing a part 20 of a body of a user when the electronic device 101 is worn by the user and a second side 212 opposite to the first side 211. The electronic device 101 may include a sensor module 250 disposed between the first side 211 and the second side 212 and including a light emitting unit 251 and a light receiving unit 252 spaced apart from the light emitting unit 251. The housing 210 may include a groove 261 disposed between the light emitting unit 251 and the light receiving unit 252 and dent toward the second side 212 from the first side 211.

According to an embodiment, the housing 210 may further include at least one protrusion 410 protruding from the first side 211 toward the part 20 of the body of the user on which the electronic device 101 is worn. The at least one protrusion 410 may include a first protrusion 411 on the light emitting unit 251 and a second protrusion 412 on the light receiving unit 252.

For example, the at least one protrusion 410 may protrude toward a center (e.g., the center C of FIG. 2A) of a hole 270. For example, in a case that the electronic device 101 is worn by the user, the at least one protrusion 410 may have a convex shape toward the part 20 of the body of the user on which the electronic device 101 is worn. The at least one protrusion 410 may have a dome shape, but is not limited thereto. For example, the at least one protrusion 410 may be in contact with the part 20 of the body of the user on which the electronic device 101 is worn.

For example, the first protrusion 411 may protrude from a portion of the first side 211 disposed on the light emitting unit 251. For example, the first protrusion 411 may be disposed between the light emitting unit 251 and the part 20 of the body of the user on which the electronic device 101 is worn. For example, when the first protrusion 411 is viewed from above (e.g., a z-axis direction), the first protrusion 411 may overlap the light emitting unit 251. For example, the first protrusion 411 may include a curved surface 411a bent with a curvature. The curved surface 411a may be an interface at which a medium is different based on the curved surface 411a. Since the first protrusion 411 has the curved surface 411a having the curvature, light emitted from the light emitting unit 251 toward the part 20 of the body of the user on which the electronic device 101 is worn may be refracted when passing through the first protrusion 411. The first protrusion 411 may diffuse the light emitted from the light emitting unit 251 toward the part 20 of the body by refracting the light. The first protrusion 411 may increase an amount of the light emitted from the light emitting unit 251 to be transmitted to the part 20 of the body, by diffusing the light.

For example, the second protrusion 412 may protrude from a portion of the first side 211 disposed on the light receiving unit 252. For example, the second protrusion 412 may be disposed between the light receiving unit 252 and the part 20 of the body of the user on which the electronic device 101 is worn. For example, the second protrusion 412 may overlap the light receiving unit 252 when the second protrusion 412 is viewed from above (e.g., the z-axis direction). For example, the second protrusion 412 may include a curved surface 412a bent with a curvature. The curved surface 412a may be the interface at which a medium is different based on the curved surface 412a. Since the second protrusion 412 has the curved surface 412a having the curvature, light emitted from the light emitting unit 251 and reflected by the part 20 of the body of the user may be refracted when passing through the second protrusion 412. The second protrusion 412 may concentrate the light reflected by the part 20 of the body toward the light receiving unit 252 by refracting the light. By concentrating the light, the second protrusion 412 may increase an amount of the light reflected by the part 20 of the body to be transmitted to the light receiving unit 252.

According to an embodiment, the sensor module 250 may further include another light receiving unit 253. At least one protrusion 410 may include a third protrusion 413 on the other light receiving unit 253. The third protrusion 413 may be formed substantially the same as or similar to the second protrusion 412 on the light receiving unit 252. For example, by including a curved surface 413a, the third protrusion 413 may concentrate the light emitted from the light emitting unit 251 and reflected by the part 20 of the body of the user on which the electronic device 101 is worn toward the other light receiving unit 253. By concentrating the light, the third protrusion 413 may increase an amount of the light reflected by the part 20 of the body to be transmitted to the other light receiving unit 253.

According to an embodiment, a distance d3 from the second side 212 to the first side 211 may be longer than a distance d4 from the second side 212 to the groove 261, and be shorter than a distance d5 from the second side 212 to the at least one protrusion 410. For example, the distance d3 from the second side 212 to the first side 211 may be longer than the distance d4 from the second side 212 to a point of an inner surface of the groove 261. For example, the distance d3 from the second side 212 to the first side 211 may be shorter than the distance d5 from the second side 212 to a point of the curved surfaces 411a, 412a, and 413a of the at least one protrusion 410. Since the distance d3 is longer than the distance d4, the electronic device 101 may reduce cross talk as light emitted from the light emitting unit 251 toward the light receiving unit 252 is received by the light receiving unit 252. Since the distance d3 is shorter than the distance d5, the electronic device 101 may increase an amount of light emitted from the light emitting unit 251 toward the part 20 of the body of the user on which the electronic device 101 is worn and reflected by the part 20 of the body to be transmitted to the light receiving unit 252 (and/or the other light receiving unit 253).

According to an embodiment, the electronic device 101 may include at least one shielding member 420 between the first side 211 and the second side 212. The at least one shielding member 420 may include a first shielding member 421 disposed between the light emitting unit 251 and the light receiving unit 252 to reduce transmission to the light receiving unit 252 of the light emitted from the light emitting unit 251 toward the light receiving unit 252. According to an embodiment, the at least one shielding member 420 may further include a second shielding member 422 disposed between the light emitting unit 251 and the other light receiving unit 253 to reduce transmission to the other light receiving unit 253 of the light emitted from the light emitting unit 251 toward the other light receiving unit 253.

According to an embodiment, the at least one shielding member 420 may protrude, in the housing 210, toward the first side 211. For example, the at least one shielding member 420 may separate the light emitting unit 251 and the light receiving unit 252. The at least one shielding member 420 may separate the light emitting unit 251 and the other light receiving unit 253. For example, together with the groove 261, the first shielding member 421 may be disposed on a path of the light emitted from the light emitting unit 251 toward the light receiving unit 252. By reflecting the light transmitted to the first shielding member 421, the first shielding member 421 may reduce cross talk as the light receiving unit 252 receives light not reflected by the part 20 of the body of the user, together with the groove 261 configured to refract light. The second shielding member 422 may be disposed on the path of the light emitted from the light emitting unit 251 toward the other light receiving unit 253, together with the other groove 262. The second shielding member 422 may reduce cross talk as the other light receiving unit 253 receives light not reflected by the part 20 of the body, together with the other groove 262 configured to refract light.

By including the at least one protrusion 410, the electronic device 101 according to the above-described embodiment includes the at least one protrusion 410 to increase the amount of the light emitted from the light emitting unit 251 to be transmitted to the part 20 of the body of the user and increase the amount of the light reflected by the part 20 of the body to be transmitted to the light receiving unit 252 (and/or the other light receiving unit 253). By including the at least one shielding member 420 configured to reflect light, the electronic device 101 may reduce the cross talk as the light receiving unit 252 and/or the other light receiving unit 253 receive the light not reflected by the part 20 of the body.

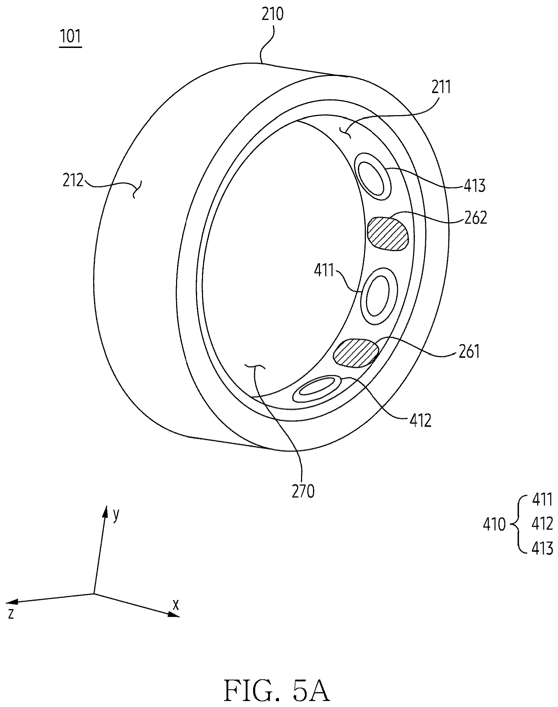

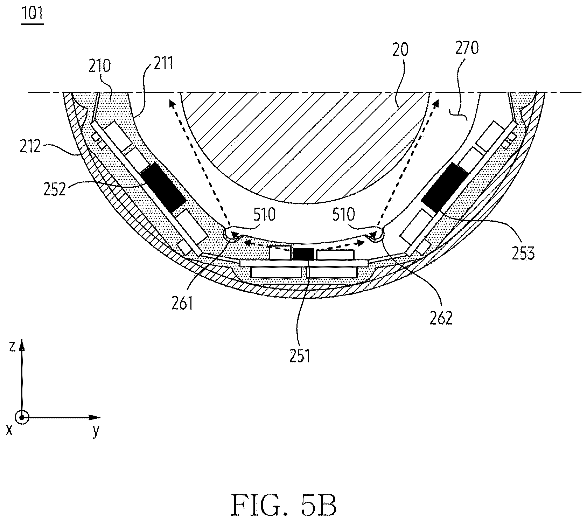

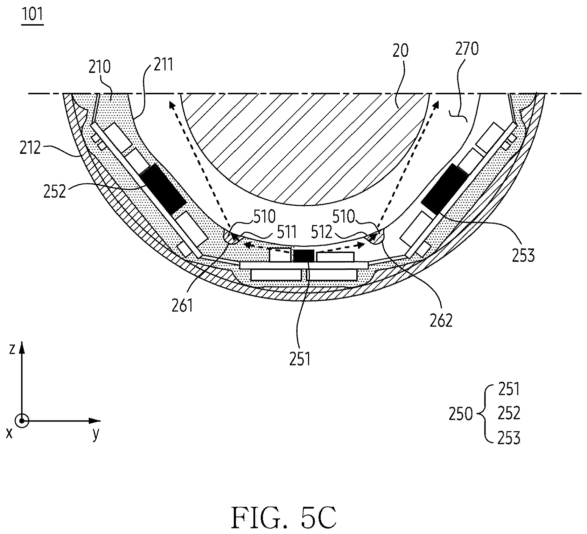

FIG. 5A is a perspective view of an exemplary electronic device. FIGS. 5B and 5C are partial cross-sectional views of the exemplary electronic device of FIG. 5A.

Referring to FIGS. 5A, 5B, and 5C, an electronic device 101 may include a housing 210 including a first side 211 facing a part (e.g., the part 20 of the body of FIG. 2A) of a body of a user when the electronic device 101 is worn by the user, and a second side 212 opposite to the first side 211. The electronic device 101 may include a sensor module 250 disposed between the first side 211 and the second side 212 and including a light emitting unit 251 and a light receiving unit 252 spaced apart from the light emitting unit 251. The housing 210 may include a groove 261 disposed between the light emitting unit 251 and the light receiving unit 252 and dent toward the second side 212 from the first side 211.

According to an embodiment, the electronic device 101 may further include a deposition material 510 disposed in the groove 261 to cover an inner surface of the groove 261, and refracting light when the light emitted from the light emitting unit 251 passes toward the groove 261.

For example, the deposition material 510 may be configured with a material different from the housing 210 forming the first side 211 and/or an element (e.g., the first frame 220 of FIG. 2B) of the housing 210. For example, the deposition material 510 may be coated on the inner surface of the groove 261. For example, the deposition material 510 may occupy at least a portion of an inner space of the groove 261.

For example, since the groove 261 is disposed on a path of light emitted from the light emitting unit 251 toward the light receiving unit 252, the light may be transmitted to the deposition material 510 covering the inner surface of the groove 261. Since a medium through which the light is transmitted is different based on the inner surface of the groove 261, the light may be refracted by the deposition material 510. According to an embodiment, the deposition material 510 may absorb a portion of the light transmitted to the deposition material 510. The electronic device 101 may reduce cross talk as light not reflected by the part 20 of the body of the user on which the electronic device 101 is worn is received by the light receiving unit 252, by including the deposition material 510 covering the inner surface of the groove 261.

According to an embodiment, the sensor module 250 may further include another light receiving unit 253. The housing 210 may include another groove 262 between the light emitting unit 251 and the other light receiving unit 253. The deposition material 510 may cover an inner surface of the other groove 262 to reduce transmission of light from the light emitting unit 251 to the other light receiving unit 253.

According to an embodiment, the deposition material 510 may occupy the groove 261 so that an exposed side of the deposition material 510 is continuous. For example, the deposition material 510 may fill the inner space of the groove 261. The deposition material 510 may fill an inner space of the other groove 262. As the deposition material 510 fills the groove 261 and the other groove 262, the deposition material 510 may include a third side 511 and a fourth side 512 exposed to the outside. Since the deposition material 510 occupies the groove 261 and the other groove 262, each of the third side 511 and the fourth side 512 may extend from the first side 211 to be continuous with the first side 211. For example, a distance from the second side 212 to the first side 211 and a distance from the second side 212 to the third side 511 (and/or the fourth side 512) may be substantially the same. Since each of the third side 511 and the fourth side 512 extends from the first side 211, the electronic device 101 may reduce foreign substances from entering the inside of the groove 261 and/or the other groove 262 from the outside.

The electronic device 101 according to the above-described embodiment may include the deposition material 510 covering the inner surface of the groove 261 and/or the other groove 262, thereby reducing transmission of light emitted from the light emitting unit 251 and not reflected by the part 20 of the body of the user to the light receiving unit 252 and/or the other light receiving unit 253. The deposition material 510 may reduce the foreign substances from entering into the inside of the groove 261 and/or the other groove 262 from the outside by occupying the groove 261 and/or the other groove 262.

A wearable device (e.g., the electronic device 101 of FIG. 1) according to the above-described embodiment may include a housing (e.g., the housing 210 of FIG. 2A) including a first side (e.g., the first side 211 of FIG. 2A) facing a part of a body (e.g., the part 20 of the body of FIG. 2A) of a user when the wearable device is worn by the user, and a second side (e.g., the second side 212 of FIG. 2A) opposite to the first side. The wearable device may include a sensor module (e.g., the sensor module 250 of FIG. 2B) disposed between the first side and the second side, the sensor module including a light emitting unit (e.g., the light emitting unit 251 of FIG. 2B) and a light receiving unit (e.g., the light receiving unit 252 of FIG. 2B) spaced apart from the light emitting unit. The housing may include a groove (e.g., the groove 261 of FIG. 2C) disposed between the light emitting unit and the light receiving unit and dent toward the second side from the first side. According to the above-mentioned embodiment, the electronic device may reduce cross talk as light emitted from the light emitting unit and not reflected by the part of the body is received by the light receiving unit by including the groove. The above-mentioned embodiment may have various effects including the above-mentioned effects.

According to an embodiment, the housing may further include a first frame (e.g., the first frame 220 of FIG. 2B) defining the first side and contacting the part of the body of the user, and a second frame (e.g., the second frame 230 of FIG. 2B) defining the second side and coupled with the first frame. According to the above-mentioned embodiment, the electronic device may be configured to improve wearability of the user and fasten the electronic device to the part of the body of the user in a case that the user wears the electronic device by including the first frame and the second frame. The above-mentioned embodiment may have various effects including the above-mentioned effects.

The wearable device according to an embodiment may further include a flexible printed circuit board (e.g., the flexible printed circuit board 240 of FIG. 2B) disposed between the first side and the second side and connected to the sensor module. The flexible printed circuit board may include a first support portion (e.g., the first support portion 241 of FIG. 2B) supporting the light emitting unit, a second support portion (e.g., the second support portion 242 of FIG. 2B) spaced apart from the first support portion and supporting the light receiving unit, and a connection portion (e.g., the first connection portion 245a of FIG. 2B) connecting the first support portion and the second support portion. According to the above-mentioned embodiment, the electronic device may connect a plurality of electronic components inside the electronic device to each other by including the flexible printed circuit board. The above-mentioned embodiment may have various effects including the above-mentioned effects.

According to an embodiment, an inner surface of the groove may include a curved surface (e.g., the curved surface 261a of FIG. 2C) extending from the first side and bending to have a curvature. According to the above-mentioned embodiment, the groove may reduce cross talk as light emitted from the light emitting unit toward the light receiving unit is received by the light receiving unit, by including the curved surface. The above-mentioned embodiment may have various effects including the above-mentioned effects.

According to an embodiment, the inner surface of the groove may include at least one inclined surface (e.g., the first inclined surface 261b and the second inclined surface 261c of FIG. 3A) extending from the first side and inclined with respect to an edge of the groove. According to the above-mentioned embodiment, the groove may reduce the cross talk as the light emitted from the light emitting unit toward the light receiving unit is received by the light receiving unit by including the at least one inclined surface. The above-mentioned embodiment may have various effects including the above-mentioned effects.

According to an embodiment, the housing may further include at least one protrusion (e.g., the at least one protrusion 410 of FIG. 4A) protruding from the first side toward the part of the body of the user wearing the wearable device. The at least one protrusion may include a first protrusion (e.g., the first protrusion 411 of FIG. 4A) on the light emitting unit and a second protrusion (e.g., the second protrusion 412 of FIG. 4A) on the light receiving unit. According to the above-mentioned embodiment, the electronic device may increase an amount of the light emitted from the light emitting unit toward the part of the body of the user to reach the part of the body, and increase an amount of the light reflected by the part of the body to be transmitted to the light receiving unit, by including the at least one protrusion. The above-mentioned embodiment may have various effects including the above-mentioned effects.

According to an embodiment, a distance (e.g., the distance d3 of FIG. 4A) from the second side to the first side may be longer than a distance (e.g., the distance d4 of FIG. 4A) from the second side to the groove, and be shorter than a distance (e.g., the distance d5 of FIG. 4A) from the second side to the at least one protrusion. According to the above-mentioned embodiment, since the distance d3 is longer than the distance d4, the electronic device may reduce cross talk as the light receiving unit receives light not reflected by the part of the body of the user. Since the distance d3 is shorter than the distance d5, the electronic device may increase the amount of the light emitted from the light emitting unit toward the part of the body of the user to reach the part of the body, and increase the amount that the light reflected by the part of the body to be transmitted to the light receiving unit. The above-mentioned embodiment may have various effects including the above-mentioned effects.

The wearable device according to an embodiment may further include a hole (e.g., the hole 270 of FIG. 2A) formed by the first side to pass through the part of the body of the user when the wearable device is worn by the user. A distance (e.g., the distance d1 of FIG. 2A) from a center (e.g., the center C of FIG. 2A) of the hole to the first side may be shorter than a distance (e.g., the distance d2 of FIG. 2A) from the center to the groove. According to the above-mentioned embodiment, since the distance d1 is shorter than the distance d2, the electronic device may reduce the cross talk as the light receiving unit receives the light not reflected by the part of the body of the user. The above-mentioned embodiment may have various effects including the above-mentioned effects.

According to an embodiment, an angle (e.g., the angle a1 of FIG. 2C) between a first direction (e.g., the first direction 281 of FIG. 2C) from the light emitting unit toward the center and a second direction (e.g., the second direction 282 of FIG. 2C) from the light receiving unit toward the center may be within a range of 45 degrees or more and 55 degrees or less. According to the above-mentioned embodiment, as the angle a1 is within the range of 45 degrees or more and 55 degrees or less, the electronic device may increase an amount of light emitted from the light emitting unit and reflected by the part of the body of the user to be transmitted to the light receiving unit. The above-mentioned embodiment may have various effects including the above-mentioned effects.

The wearable device according to an embodiment may further include a shielding member (e.g., the first shielding member 411 of FIG. 4A) between the first side and the second side, disposed between the light emitting unit and the light receiving unit to reduce transmission to the light receiving unit of light emitted from the light emitting unit toward the light receiving unit. According to the above-mentioned embodiment, the electronic device may reduce the cross talk as the light emitting unit receives the light not reflected by the part of the body of the user, by including the shielding member. The above-mentioned embodiment may have various effects including the above-mentioned effects.

The wearable device according to an embodiment may further include a deposition material (e.g., the deposition material 510 of FIG. 5B) disposed in the groove to cover an inner surface of the groove, and refracting light when the light emitted from the light emitting unit passes toward the groove. According to the above-mentioned embodiment, the electronic device may reduce the cross talk as the light emitting unit receives the light not reflected by the part of the body of the user, by including the deposition material. The above-mentioned embodiment may have various effects including the above-mentioned effects.

According to an embodiment, the deposition material may occupy the groove so that an exposed side (e.g., the third side 511 or the fourth side 512 of FIG. 5C) of the deposition material is continuous with the first side. According to the above-mentioned embodiment, the deposition material may reduce foreign substances from entering the inside of the groove from the outside, by occupying the groove. The above-mentioned embodiment may have various effects including the above-mentioned effects.

According to an embodiment, the sensor module may further include another light receiving unit (e.g., the other light receiving unit 253 of FIG. 2B) that is spaced apart from the light emitting unit and faced away from the light receiving unit. The housing may further include another groove (e.g., the other groove 262 of FIG. 2C) disposed between the light emitting unit and the another light receiving unit, and dent toward the second side from the first side. According to the above-mentioned embodiment, the electronic device may reduce cross talk as the another light receiving unit receives the light not reflected by the part of the body of the user, by including the other light receiving unit and the another groove. The above-mentioned embodiment may have various effects including the above-mentioned effects.

The wearable device according to an embodiment may further include a hole formed by the first side to pass through the part of the body of the user when the wearable device is worn by the user. An angle between a first direction from the light emitting unit toward a center of the hole and a second direction from the light receiving unit toward the center may correspond to an angle (e.g., the angle a2 of FIG. 2A) between the first direction from the light emitting unit toward the center and a third direction (e.g., the third direction 283 of FIG. 2A) from the other light receiving unit toward the center. According to the above-mentioned embodiment, as the angle a1 corresponds to the angle a2, the electronic device may increase an amount of the light emitted from the light emitting unit and reflected by the part of the body of the user to be transmitted to the light receiving unit and the another light receiving unit. The above-mentioned embodiment may have various effects including the above-mentioned effects.

The wearable device according to an embodiment may further include a processor (e.g., the processor 120 of FIG. 1) operatively coupled to the sensor module. The processor may be configured to emit light toward the part of the body of the user on which the wearable device is worn, using the light emitting unit of the sensor module. The processor may be configured to obtain biometric information of the user by receiving at least a portion of the light reflected by the part of the body using the light receiving unit of the sensor module. According to the above-mentioned embodiment, the electronic device may provide the biometric information of the user to the user of the electronic device using the sensor module, by including the processor operatively coupled to the sensor module. The above-mentioned embodiment may have various effects including the above-mentioned effects.

A wearable device according to an embodiment may include a housing including a first side facing a part of a body of a user when the wearable device is worn by the user, and a second side opposite to the first side. The wearable device may include a hole formed by the first side to pass through the part of the body of the user. The wearable device may include a sensor module disposed between the first side and the second side, the sensor module including a light emitting unit and a light receiving unit spaced apart from the light emitting unit. The wearable device may include a processor operatively coupled to the sensor module. The housing may include a groove disposed between the light emitting unit and the light receiving unit and dent toward the second side from the first side to reduce transmission to the light receiving unit of light emitted from the light emitting unit toward the light receiving unit. The processor may be configured to emit light toward the part of the body of the user on which the wearable device is worn, using the light emitting unit of the sensor module, and obtain biometric information of the user by receiving at least a portion of the light reflected by the part of the body using the light receiving unit of the sensor module. According to the above-mentioned embodiment, the electronic device may reduce cross talk as light emitted from the light emitting unit and not reflected by the part of the body is received by the light receiving unit, by including the groove. The electronic device may provide the biometric information of the user to the user of the electronic device using the sensor module, by including the processor operatively coupled to the sensor module. The above-mentioned embodiment may have various effects including the above-mentioned effects.

According to an embodiment, an inner surface of the groove may include a curved surface extending from the first side and bending to have a curvature. According to the above-mentioned embodiment, the groove may reduce cross talk as light emitted from the light emitting unit toward the light receiving unit is received by the light receiving unit, by including the curved surface. The above-mentioned embodiment may have various effects including the above-mentioned effects.

According to an embodiment, the housing may further include at least one protrusion protruding from the first side toward the part of the body of the user wearing the wearable device. The at least one protrusion may include a first protrusion on the light emitting unit and a second protrusion on the light receiving unit. According to the above-mentioned embodiment, the electronic device may increase an amount of light emitted from the light emitting unit toward the part of the body of the user to reach the part of the body, and increase an amount of the light reflected by the part of the body to be transmitted to the light receiving unit by including the at least one protrusion. The above-mentioned embodiment may have various effects including the above-mentioned effects.

The wearable device according to an embodiment may further include a shielding member between the first side and the second side, disposed between the light emitting unit and the light receiving unit to reduce transmission to the light receiving unit of light emitted from the light emitting unit toward the light receiving unit. According to the above-mentioned embodiment, the electronic device may reduce the cross talk as the light emitting unit receives the light not reflected by the part of the body of the user, by including the shielding member. The above-mentioned embodiment may have various effects including the above-mentioned effects.

The wearable device according to an embodiment may further include a deposition material disposed in the groove to cover an inner surface of the groove, and refracting light when the light emitted from the light emitting unit passes toward the groove. According to the above-mentioned embodiment, the electronic device may reduce the cross talk as the light emitting unit receives the light not reflected by the part of the body of the user, by including the deposition material. The above-mentioned embodiment may have various effects including the above-mentioned effects.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (101) comprising:
a housing (210) including a first side (211) facing a part (20) of a body of a user when the wearable device (101) is worn by the user, and a second side (212) opposite to the first side (211); and
a sensor module (176; 250) disposed between the first side (211) and the second side (212), the sensor module comprising a light emitting unit (251) and a light receiving unit (252) spaced apart from the light emitting unit (251);
wherein the housing (210) includes a groove (261) disposed between the light emitting unit (251) and the light receiving unit (252) and dent toward the second side (212) from the first side (211).

2. The wearable device (101) of claim 1,
wherein the housing (210) further comprises:
a first frame (220) defining the first side (211) and contacting the part (20) of the body of the user; and
a second frame (230) defining the second side (212) and coupled with the first frame (220).

3. The wearable device (101) of claim 1 or 2, further comprising:
a flexible printed circuit board (240) disposed between the first side (211) and the second side (212) and connected to the sensor module (176; 250),
wherein the flexible printed circuit board (240) includes:
a first support portion (241) supporting the light emitting unit (251);
a second support portion (242) spaced apart from the first support portion (241) and supporting the light receiving unit (252); and
a connection portion (245a) connecting the first support portion (241) and the second support portion (242).

4. The wearable device (101) of any one of claims 1 to 3,
wherein an inner surface of the groove (261) includes a curved surface (261a) extending from the first side (211) and bending to have a curvature.

5. The wearable device (101) of any one of claims 1 to 4,
wherein the inner surface of the groove (261) includes at least one inclined surface (261b; 261c) extending from the first side (211) and inclined with respect to an edge of the groove (261).

6. The wearable device (101) of any one of claims 1 to 5,
wherein the housing (210) further comprises at least one protrusion (410; 411; 412; 413) protruding from the first side (211) toward the part (20) of the body of the user wearing the wearable device (101), and
wherein the at least one protrusion (410; 411; 412; 413) includes a first protrusion (411) on the light emitting unit (251) and a second protrusion (412) on the light receiving unit (252).

7. The wearable device (101) of any one of claims 1 to 6,
wherein a distance (d3) from the second side (212) to the first side (211) is longer than a distance (d4) from the second side (212) to the groove (261), and is shorter than a distance (d5) from the second side (212) to the at least one protrusion (410; 411; 412; 413).

8. The wearable device (101) of any one of claims 1 to 7, further comprising:
a hole (270) formed by the first side (211) to pass through the part (20) of the body of the user when the wearable device (101) is worn by the user,
wherein a distance (d1) from a center (C) of the hole (270) to the first side (211) is shorter than a distance (d2) from the center (C) to the groove (261).

9. The wearable device (101) of any one of claims 1 to 8,
wherein an angle (a1) between a first direction (281) from the light emitting unit (251) toward the center (C) and a second direction (282) from the light receiving unit (252) toward the center (C) is within a range of 45 degrees or more and 55 degrees or less.

10. The wearable device (101) of any one of claims 1 to 9, further comprising:
a shielding member (421) between the first side (211) and the second side (212), disposed between the light emitting unit (251) and the light receiving unit (252) to reduce transmission to the light receiving unit (252) of light emitted from the light emitting unit (251) toward the light receiving unit (252).

11. The wearable device (101) of any one of claims 1 to 10, further comprising:
a deposition material (510) disposed in the groove (261) to cover an inner surface of the groove (261), and refracting light when the light emitted from the light emitting unit (251) passes toward the groove (261).

12. The wearable device (101) of any one of claims 1 to 11,
wherein the deposition material (510) occupies the groove (261) so that an exposed side (511) of the deposition material (510) is continuous with the first side (211).

13. The wearable device (101) of any one of claims 1 to 12,
wherein the sensor module (176; 250) further comprises another light receiving unit (253) that is spaced apart from the light emitting unit (251) and faced away from the light receiving unit (252), and
wherein the housing (210) further comprises another groove (262) disposed between the light emitting unit (251) and the another light receiving unit (253), and dent toward the second side (212) from the first side (211).

14. The wearable device (101) of any one of claims 1 to 13, further comprising:
a hole (270) formed by the second side (212) to pass through the part (20) of the body of the user when the wearable device (101) is worn by the user,
wherein an angle (a1) between a first direction (281) from the light emitting unit (251) toward a center (C) of the hole (270) and a second direction (282) from the light receiving unit (252) toward the center (C) corresponds to an angle (a2) between the first direction (281) from the light emitting unit (251) toward the center (C) and a third direction (283) from the another light receiving unit (253) toward the center (C).

15. The wearable device (101) of any one of claims 1 to 14, further comprising a processor (120) operatively coupled to the sensor module (176; 250),
wherein the processor (120) is configured to:
emit light toward the part (20) of the body of the user on which the wearable device (101) is worn, using the light emitting unit (251) of the sensor module (176; 250), and
obtain biometric information of the user by receiving at least a portion of the light reflected by the part (20) of the body using the light receiving unit (252) of the sensor module (176; 250).
